Europäisches Patentamt

European Patent Office    (11) Publication number:    **0 136 765**

Office européen des brevets    **A2**

(12)    # EUROPEAN PATENT APPLICATION

(21) Application number: **84201422.7**    (51) Int. Cl.⁴: **C 07 C 126/02**

(22) Date of filing: **05.10.84**

(30) Priority: **06.10.83 NL 8303425**

(43) Date of publication of application:
**10.04.85 Bulletin 85/15**

(84) Designated Contracting States:
**AT BE DE FR GB IT NL**

(71) Applicant: **UNIE VAN KUNSTMESTFABRIEKEN B.V.**
**Maliebaan 81**
**NL-3581 CG Utrecht(NL)**

(72) Inventor: **Van Nassau, Petrus Johannes Marie**
**Burg. Pylsstraat 4**
**NL-6151 HA Munstergeleen(NL)**

(74) Representative: **Roeffen, Wilhelmus Johannes**
**Maria et al,**
**OCTROOIBUREAU DSM Postbus 9**
**NL-6160 MA Geleen(NL)**

(54) Process for the preparation of urea.

(57) An improved process for the preparation of urea from
carbon dioxide and ammonia at elevated pressure and
temperature in at least one reaction zone to form a urea
synthesis solution containing non-converted ammonium
carbamate and free ammonia. At least a part of the ammonia
and carbon dioxide are reacted to form ammonium carba-
mate and urea in a first reaction zone adapted to exchange
heat with a first stripping zone so that the heat produced by
the condensation of ammonia and carbon dioxide in the first
reaction zone is exchanged with urea synthesis solution in
the first stripping zone so as to decompose a portion of the
ammonium carbamate contained therein. A gas mixture
containing ammonia and carbon dioxide is removed from
the first stripping zone and recycled, after at least partial
condensation and absorption in a condensation zone, to be
used in the formation of a further amount of urea synthesis
solution. The stripped urea synthesis solution, still contain-
ing some residual ammonium carbamate, is reduced in
pressure and introduced into a decomposition zone ex-
changing heat with the condensation zone in a manner such
that heat released by the condensation is exchanged with the
stripped urea solution in the decomposition zone whereby at
least a portion of residual ammonium carbamate in the
stripped urea solution is decomposed.

FIG 1

## PROCESS FOR THE PREPARATION OF UREA

### Background of the Invention

This invention relates to a process for the preparation of urea.

One process for the preparation of urea which has found wide acceptance is described in European Chemical News Urea Supplement of 17th January, 1969, pages 17-20. In that process a urea synthesis solution formed in a reaction zone at high pressure and temperature is subjected to a stripping treatment at the synthesis pressure. In this stripping treatment, the synthesis solution is heated and contacted countercurrently with gaseous carbon dioxide to decompose a major portion of the carbamate contained therein into ammonia and carbon dioxide. These decomposition products are expelled from the solution and, together with a small quantity of water vapor and the carbon dioxide used for stripping, are separated from the remaining urea product stream as a gas mixture. This gas mixture is subsequently partially condensed in a condensation zone to form an aqueous ammonium carbamate solution which, together with the remaining non-condensed gas mixture, is returned to the reaction zone for the formation of a further quantity of urea. The further condensation of this remaining gas mixture in the reactor provides the heat required for the conversion of ammonium carbamate into urea, and no additional heat need be supplied to the reactor from outside.

The heat required for this stripping treatment is obtained by condensation of high-pressure steam on the outside of the tubes of a vertical heat exchanger in which the stripping takes place. According to this publication, about 1000 kg of about 25 bar steam is required per ton of urea. In practice, however, this consumption of high-pressure steam (25 bar) has now been reduced to about 850 kg per ton urea. Moreover, it is possible to partially recover the heat used in the stripping treatment by condensing the gas mixture which has been separated off, but in view of the relatively low temperature at which this condensation takes place, only steam of 3-5 bar can be formed, which often cannot effectively be used either within or outside of the process. For this reason, and particularly in view of the continually increasing energy prices, the consumption of high-pressure steam must be reduced as far as possible and, depending on local conditions, it may be desirable to avoid or strongly reduce the production of surplus low-pressure steam.

Various proposals have already been made to use the heat released in the condensation of ammonia and carbon dioxide into ammonium carbamate to provide or at least supplement the heat requirements of the stripping treatment.

One such proposal is disclosed in British patent application 2,107,704 wherein ammonium carbamate and urea formation take place in the shell side of a vertical tubular heat exchanger which serves as a reaction zone, and the urea formation continues in an after-reaction zone. The pressure in the shell side of the vertical tubular heat exchanger is preferably about 20 to 120 bar higher than the pressure within the tubes. At least a portion of the urea synthesis solution formed in the after-reaction zone is subjected to a stripping treatment in the tubes of the vertical tubular heat exchanger, and the heat required for this stripping is supplied by a portion of the heat of carbamate formation released in the shell side. By having part of the urea formation take place in the shell side reaction zone, the average temperature there is substantially increased, as a result of which the heat of reaction becomes available at such a high temperature level that an acceptable stripping efficiency can be achieved without the necessity of high-pressure steam.

In one embodiment of that patent shown in Figure 4, two stripping stages are employed in parallel wherein only a portion of the urea synthesis solution produced in the after-reaction zone is stripped using the heat of carbamate formation, without the use of an external stripping gas, and the remainder is treated in a heater-decomposer using steam. In all of the embodiments there disclosed, however, condensation of the gas mixtures containing ammonia and carbon dioxide that are obtained on stripping yields substantial amounts of low-pressure steam of 3 to 5 bar, for which there is little use either inside or outside of the urea process.

It has also been proposed in U.S. patent No. 4,354,040 to utilize the heat released in the condensation of the gas mixture containing the ammonia and carbon dioxide obtained in stripping a urea synthesis solution, in part for generation of low-pressure steam, and in part as a heat source for a high-pressure carbamate decomposer operating at 12 to 25 bar wherein further amounts of ammonium carbamate contained in the stripped urea synthesis solution are decomposed. In that process the high-pressure carbamate condenser is constructed as two separate parts divided by tubesheets, one on top of the other. In the shell-side space of the top part, the stripped urea synthesis solution, expanded to 12 to 25 bar, is passed in indirect heat exchange with ammonium carbamate condensing in the tubes from the ammonia and carbon dioxide gases obtained in the stripping. This condensation to ammonium carbamate is then completed in the tubes of the bottom part of this apparatus, with the heat of condensation thus released being carried off by heat exchange with water, to form low-pressure steam. Apart from the need for an additional high-pressure decomposition stage requiring high-pressure steam, the process there described has the disadvantage that the high-pressure carbamate condenser is of a complex construction, and thus is expensive. These disadvantages are only in part eliminated by the embodiment disclosed in British patent application 2,109,372, in which use is made of two separate high-pressure carbamate condensers rather than one high-pressure carbamate condenser consisting of two parts. Nevertheless, in the high-pressure decomposition stage of these known processes, low-pressure steam is generated on condensation of the gas mixtures obtained in the stripping treatment.

The object of the present invention is to provide a process for the preparation of urea in which the supply of high-pressure steam in the reaction or synthesis section is substantially reduced or can even be dispensed with, while the formation of low-pressure steam can be greatly reduced or entirely eliminated. According to the invention, this is achieved by a combination of measures as a result of which the heat required for the decomposition and stripping of ammonium carbamate from the urea synthesis solution is, at least in part, provided by the heat of condensation of ammonia and carbon dioxide into ammonium carbamate in a reaction zone by means of indirect heat exchange between at least a portion of the urea synthesis solution and ammonium carbamate formed in the reaction zone, while moreover the heat released on condensation of the gas mixtures obtained in this stripping treatment is utilized for decomposition of further amounts of ammonium carbamate still present in the stripped urea synthesis solution. As used herein, the term indirect heat exchange should be understood to mean the transfer of heat between two components across a heat exchange surface, such as the wall of a heat exchanger tube, rather than by direct mixing of the two components, on the one hand, or via the intermediate production of steam, on the other.

The invention, therefore, relates to a process for the preparation of urea in which a urea synthesis solution containing non-converted ammonium carbamate and free ammonia is formed at high pressure and temperature from carbon dioxide and an excess of ammonia, wherein the reaction between ammonia and carbon dioxide is at least in part effected in a reaction zone adapted to permit an exchange of heat with a urea synthesis solution being treated in a stripping zone so that the heat produced by the formation of carbamate in the reaction zone is utilized for the decomposition of non-converted carbamate in the stripping zone; wherein the gases obtained in this stripping treatment are, after at least partial condensation and absorption in a condensation zone, returned to the reaction zone; and wherein the stripped urea synthesis solution is processed into product urea such as a urea solution or solid urea. In particular, especially effective utilization of steam and efficient recovery of heat is realized, in accordance with the process of this invention, by decomposing further amounts of ammonium carbamate still present in the stripped urea synthesis solution, after pressure reduction, in a decomposition zone adapted to exchange heat with the condensation zone. In this manner, the heat of condensation produced in the condensation zone can effectively be used to decompose and remove ammonium carbamate from the urea synthesis solution, rather than to produce low pressure steam for which there is little use.

Preferably, the pressure of the stripped urea synthesis solution introduced into the decomposition zone will be reduced to the point that the pressure difference between the condensing gases in the condensation zone, and the stripped urea synthesis solution in the decomposition zone, is at least 100 bar, so that a substantial part of the carbamate still present in the stripped urea synthesis solution will be decomposed. Most preferably, the stripped urea synthesis solution will be expanded to a pressure of between about 15 and 25 bar.

The process according to the present invention can very advantageously be applied if the urea synthesis is effected as described in my copending application Serial No. _____ (corresponding to Dutch patent application not yet laid open to public inspection No. 8303424). According to the process therein described, the urea formation is at least in part effected in a first reaction zone which is adapted to exchange heat with a first stripping zone, while further urea formation is effected in a second, after-reaction zone. A portion of the urea synthesis solution from the after-reaction zone is treated in the first stripping zone and the remainder in a second stripping zone, and the stripped urea synthesis solutions thus obtained are processed into product urea. The ammonia and carbon dioxide containing gas mixture formed in the second stripping zone is condensed and absorbed into a recirculated ammonium carbamate solution in the first reaction zone in a manner such that the heat of condensation and absorption is indirectly exchanged with the urea synthesis solution in the first stripping zone to effect the decomposition and removal of ammonium carbamate as an ammonia and carbon dioxide containing gas mixture. With a sufficiently long residence time, a portion of the ammonium carbamate formed in the first reaction zone will be converted to urea, resulting in an increased temperature and a significantly greater transfer of heat to the first stripping zone. The ammonia and carbon dioxide containing gas mixture thus obtained in the first stripping zone is condensed in a condensation zone and the carbamate solution formed is recirculated to the reaction zone. In accordance with the present invention, the stripped urea synthesis solutions obtained in both stripping steps are subsequently expanded to a pressure preferably in the range of between about 15 and 25 bar and supplied to the decomposition zone adapted to exchange heat with the condensing gas mixture in the condensation zone. By means of this heat exchange, a large part of the carbamate still present in the expanded, stripped urea synthesis solutions is decomposed, and the heat of condensation released in the condensation zone is effectively removed. No separate carbamate condenser with low-pressure steam production is required. In this embodiment high-pressure steam is required exclusively in the second stripping zone.

The process according to the present invention can also be applied very suitably if the urea synthesis is effected in the way described in British patent application 1,447,914. According to the process described therein urea is prepared at a pressure of 250 to 600 atmospheres absolute, a temperature of 210 to 245°C and an ammonia to carbon dioxide molar ratio of 2.5 to 8 in a reaction zone which exchanges heat with a stripping zone. The gas mixture discharged from the stripping zone is at least in part recirculated to the reaction zone where the heat required for the stripping treatment is supplied by the formation of carbamate from the recycled gas mixture. In accordance with the present invention, by subsequently expanding the stripped urea synthesis solution to a pressure of 15 to 25 bar, and passing it in heat exchange with condensing carbamate in the tubes of the high-pressure carbamate condenser, the heat of condensation released in the high-pressure carbamate condenser can be effectively removed, and further amounts of carbamate are decomposed in the stripped urea synthesis solution. In this embodiment no high-pressure steam is required in the synthesis section for the stripping treatment, and no low-pressure steam produced in the condensation of the gases obtained in the stripping treatment.

In contrast to the processes disclosed in the above-mentioned patents, the process according to this invention does not require installation of a high-pressure carbamate decomposer; use of a gas-liquid separator and a carbamate condenser designed as heat exchanger are sufficient. Moreover, a carbamate condenser for low-pressure steam production is dispensed with. The overall result is that the investment costs are lower.

## Brief Description of the Drawings

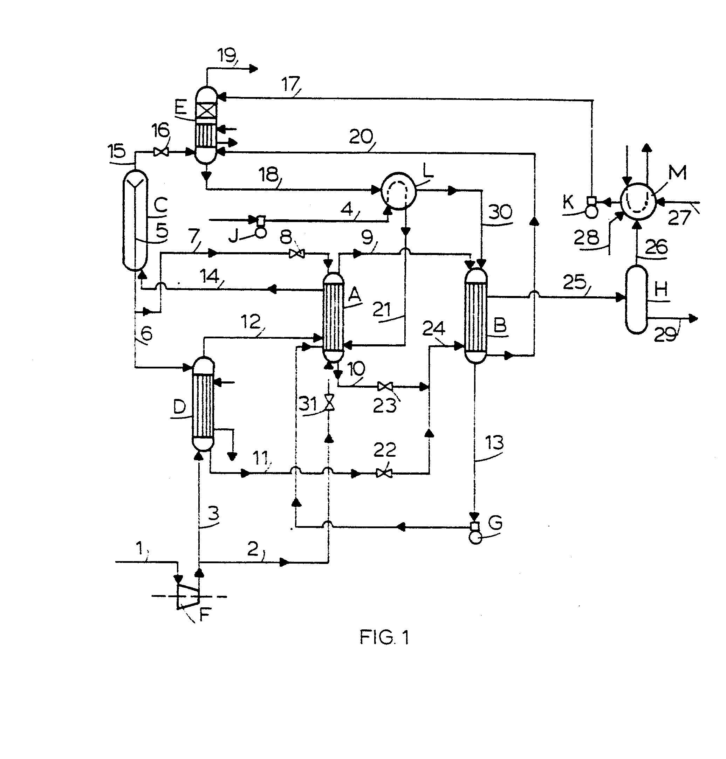

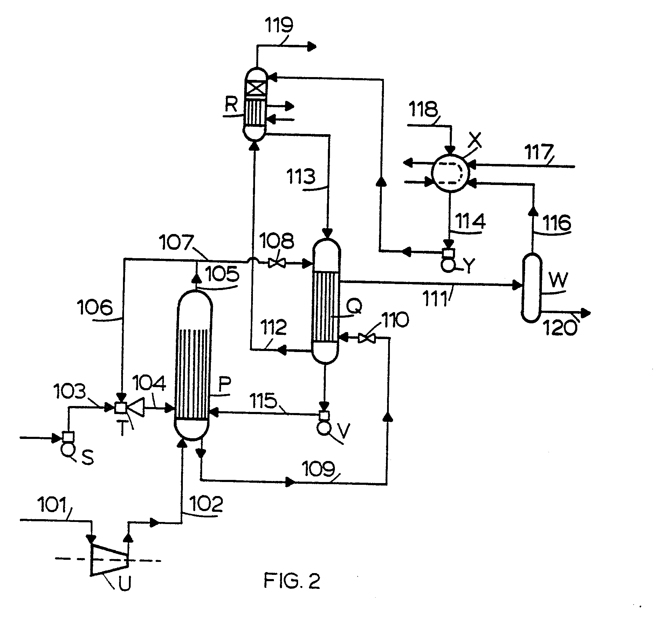

The invention will now be elucidated with reference to the drawings and the examples, without, however, being restrict thereto. Figure 1 is a schematic representation of a process in which a portion of the urea synthesis solution produced is stripped using high-pressure steam as a heat source and the remainder is stripped with the aid of the heat developed in the condensation of the gases obtained in this stripping treatment. Figure 2 shows an embodiment in which no high-pressure steam is supplied during stripping of the urea synthesis solution.

## Description of the Preferred Embodiments

In Figure 1, A denotes a reactor-stripper with the first reaction zone in the shell-side and the first stripping zone in the tubes of a vertical tubular heat exchanger. B denotes a carbamate condenser operating at high pressure, C an after-reactor constituting a second reaction zone, D a stripper constituting a second stripping zone, and E a scrubber. F and G denote, respectively, a carbon dioxide compressor and a carbamate pump. H represents a gas-liquid separator, J an ammonia pump, K a carbamate pump, L a heat exchanger, and M a medium-pressure carbamate condenser operating at, for instance, 15 to 25 bar.

The reaction zone of reactor-stripper A, after-reactor C, and stripper D, are preferably maintained at the same pressure, which can be between about 125 and 250 bar, but preferably between 175 and 210 bar. Likewise, it is preferred to have the same pressure maintained in the stripping zone of reactor-stripper A, in carbamate condenser B, and in scrubber E, which for instance must be about 20 to 60 bar lower than the pressure maintained in units A, C and D.

A urea synthesis solution is obtained in after-reactor C at a pressure of, for instance, about 175 to 210 bar, and contains, in addition to urea and water, non-converted carbamate and free ammonia. This urea synthesis solution is discharged through line 5 and split into two portions. The first portion is passed through line 6 into stripper D, where it is subjected to a stripping treatment at the same pressure as in after-reactor C. Preferably, the proportion of urea synthesis solution introduced into stripper D is between about 40 and 60 percent of the total amount. This stripping treatment is effected counter-current to gaseous carbon dioxide while heat is supplied by high-pressure steam. Carbon dioxide is supplied to this stripper through lines 1 and 3 and brought up to the required pressure of 175 to 210 bar by compressor F. Air or another oxygen-containing gas mixture is added to the carbon dioxide to keep the materials of construction coming into contact at high temperature with ammonium carbamate-containing solutions in a passive condition. The amount of carbon dioxide used in the stripping treatment in stripper D is preferably between about 30 and 50 percent of the total amount of fresh carbon dioxide required in the process.

After this stripping treatment, a stripped urea synthesis solution is discharged from stripper D through line 11, and a gas mixture consisting of the fresh carbon dioxide supplied and the ammonia, carbon dioxide, and water expelled from the urea synthesis solution by the stripping treatment is passed through line 12 to the reaction zone of reactor-stripper A. The reaction zone of reactor-stripper A is additionally fed with the ammonia required in the process which is supplied through line 4 by pump J via heat exchanger L and line 21, and with the carbamate solution formed in carbamate condensor B, which is pumped into this reaction zone through line 13 and pump G. In the reaction zone of reactor-stripper A, a major portion of the ammonia and carbon dioxide supplied through lines 12 and 4 is converted into ammonium carbamate, and this carbamate, together with the carbamate in the carbamate solution supplied through line 13, are in part converted into urea and water.

Heat is released in the formation of ammonium carbamate whereas heat is consumed in the conversion of carbamate into urea and water. There is, however, a net heat surplus which is used for the decomposition of non-converted carbamate in the stripping zone. Since the gas mixture fed to the first reaction zone through line 12 contains at least a major part of the carbon dioxide that was fed into stripper D, and since the carbamate and urea formation are effected in this reaction zone at a higher pressure than that prevailing in the stripping zone of reactor-stripper A, enough heat becomes available at a relatively high temperature level to decompose of a very substantial portion of the carbamate present in the solution supplied to this first stripping zone without the need for heat from an external source. By continuing the conversion of carbamate into urea and water in the first reaction zone until a substantial part, for instance more than half, of the equilibrium amount of urea achievable under the reaction conditions applied has been formed, a further increase in temperature is accomplished and the portion of the surplus heat transferred to the stripping zone becomes larger. This effect will be larger as equilibrium is approached. In general, therefore, the amount of urea formed will preferably be more than about 70 percent of the equilibrium value. However, urea formation in excess of 90 percent of the equilibrium amount necessitates substantially longer residence times because of the strongly decreased reaction rate, and thus requires an unattractively large volume in the reaction zone.

The higher the pressure in the first reaction zone, the higher the temperature at which the condensation of carbon dioxide and ammonia to carbamate takes place, so that more heat becomes available for carbamate decomposition in the first stripping zone. Preferably, a pressure differential of at least about 20 bar is chosen to provide a large enough temperature differential between the reaction zone and the stripping zone to ensure an adequate driving force for sufficient heat exchange. A pressure differential of more than about 120 bar is theoretically possible by requires a complex design of the plant accommodating the reaction zone and the stripping zone. Most preferably, the pressure chosen for the first reaction zone should be in the range of between about 20 and 60 bar higher than the pressure in the first stripping zone.

The first reaction zone preferably is intensively mixed so as to achieve optimum uniformity in the temperature distribution and optimum heat transfer. To a certain extent, this is already accomplished by having the gas mixture and the carbamate solution flow upwards through the reaction zone. Mixing, and thus heat transfer, is further improved by installation of baffles, guide plates, or similar elements.

The effluent from this first reaction zone, consisting of a solution of urea, water, ammonia, and ammonium carbamate and a gas mixture consisting of ammonia, carbon dioxide, water, and inerts, is passed through line 14 to after-reactor C, where urea formation is continued until at least about 90 percent of the equilibrium amount of urea achievable under the reaction conditions applied here has been formed. The required heat in after-reactor C is obtained by permitting part of the gas mixture supplied to the reaction zone of reactor-stripper A through 12 to remain uncondensed and to pass as a gas through line 14 into the after-reaction zone where it is condensed, releasing both the heat of condensation and heat of carbamate formation.

A gas mixture containing inert gases and equilibrium amounts of ammonia, carbon dioxide, and water also remains uncondensed in after-reactor C, which gas mixture is passed through line 15 and expansion valve 16 to scrubber E where, at the same pressure as in the stripping zone of reactor-stripper A and carbamate condenser B, ammonia and carbon dioxide are recovered by scrubbing with water or dilute carbamate solution supplied through line 17, and the heat of absorption is discharged. The remaining non-absorbed off-gas mixture is discharged from the system through line 19, and the relatively dilute carbamate solution obtained in scrubber E is led to carbamate condenser B through line 18, heat exchanger L, and line 30.

The remainder of the urea synthesis solution formed in after-reactor C (i.e., that portion not sent to stripper D) flows through line 7 and, after reduction of the pressure to the pressure in the first stripping zone (for instance 140 bar) via expansion valve 8, is introduced into the space above the tubes of reactor-stripper A. Here the gas mixture consisting of ammonia, carbon dioxide, and water that is released on the pressure decrease is separated from the remaining urea synthesis solution. The solution flows downward through the stripping zone along the internal walls of the tubes countercurrent to a stripping gas containing between about 50 and 70 percent of the amount of carbon dioxide required in the synthesis. The urea synthesis solution flowing downward through the tubes is heated, as a result of the heat exchange with the reaction zone, to decompose ammonium carbamate, and is stripped with the carbon dioxide. The gas mixture thus formed, together with the stripping gas supplied, flows upwardly to the space above the tubes, and is discharged, together with the gas mixture released on the pressure decrease, through line 9 and led into carbamate condenser B.

A major portion of this gas mixture is condensed in carbamate condenser B, which is also fed, via lines 18 and 30, with the carbamate solution obtained by scrubbing ammonia and carbon dioxide from the gas mixture containing ammonia, carbon dioxide, and inert gases in scrubber E. Carbamate condenser B is preferably maintained at the same pressure as the stripping zone of reactor-stripper A. The carbamate solution thereby formed is discharged through line 13, pumped up to the pressure prevailing in the reaction zone of reactor-stripper A by pump G, and introduced into that zone. The portion of the gas mixture not condensed in carbamate condenser B, consisting mainly of the inert components introduced with the carbon dioxide, is sent to scrubber E via line 20 wherein remaining ammonia and carbon dioxide are recovered.

The heat released in the condensation in carbamate condenser B is used for the decomposition of ammonium carbamate remaining in the stripped urea synthesis solution. The stripped urea solution, which has been expanded to a pressure of, for instance, 15 to 25 bar in expansion valves 22 and 23, is introduced into the shell-side space of the carbamate condenser B through line 24, and discharged therefrom through line 25. The gas-liquid mixture discharged from the shell-side space of carbamate condenser B is led through line 25 into gas-liquid separator H. The liquid phase removed from the separator, consisting of an aqueous urea solution still containing dissolved ammonia and carbon dioxide, is passed through line 29 and into a plant (not shown) for the recovery of such ammonia and carbon dioxide and for further processing into a urea solution or solid urea. The gas phase removed from gas-liquid separator H, consisting mainly of a mixture of ammonia, carbon dioxide and water vapor, is passed through line 26 into medium-pressure carbamate condenser M wherein it is condensed and absorbed into a carbamate solution, which is thereafter discharged and pumped into scrubber E by means of pump K and line 17.

In Figure 2, P denotes a reactor-stripper consisting of a cylindrical vessel with tubes mounted in a tubesheet at the bottom with the shell-side space on the top side being in open connection with the inside of the tubes. Q denotes a carbamate condenser and R a scrubber. S, T and U denote, respectively, an ammonia pump, an ejector and a carbon dioxide compressor. V represents a carbamate pump, W a gas-liquid separator, X a medium-pressure carbamate condenser and Y a carbamate pump.

At a high pressure, for instance between 245 and 350 bar, ammonium carbamate and urea are formed in the reactor part of reactor-stripper P from fresh liquid ammonia (supplied by ammonia pump S through line 103 and ejector T and line 104), a portion of the gas mixture containing ammonia and carbon dioxide obtained on stripping (aspirated by ejector T and supplied through lines 105 and 106), and a carbamate solution from carbamate condenser Q (supplied by pump V through line 115). The urea synthesis solution thus obtained is contacted in the stripper part of reactor-stripper P with carbon dioxide supplied through line 101 via carbon dioxide compressor U and line 102. The heat released in the reaction zone in the formation of carbamate is transferred to the stripping zone through the tube walls, and this heat, together with the stripping action of the carbon dioxide, serves to decompose and remove a major portion of the non-converted ammonium carbamate present in the urea synthesis solution. When there is efficient heat transfer between the reaction and stripping zone of reactor-stripper P, the surplus heat released in the carbamate formation in excess of that required for the urea formation gives a temperature in the stripping zone that is sufficiently high for reaching an acceptable stripping efficiency. Efficient heat transfer can be achieved, for instance, by promoting intensive mixing in the reaction zone.

The resulting decomposition products of ammonia and carbon dioxide, and a portion of the excess ammonia and carbon dioxide used as the stripping agent, are discharged from reactor-stripper P through line 105. A portion of this gas mixture discharged through line 105 is aspirated by ejector T through line 106 and recycled to the reaction zone through line 104. The remainder of the gas mixture obtained by stripping is passed, via line 107 and expansion valve 108, into the tube side of carbamate condenser Q wherein it is, for the most part, condensed and absorbed into a carbamate solution, obtained by scrubbing inert gases in scrubber R, fed through line 113. The pressure in carbamate condenser Q is preferably maintained in the range of between about 120 and 160 bar, for instance at 140 bar. The heat released in carbamate condenser Q on condensation of the gas mixture supplied through line 107 is used for the decomposition of further amounts of carbamate still present in the stripped urea synthesis solution supplied through line 109, and expanded in expansion valve 110 to a pressure preferably between about 15 and 25 bar, for instance 18 bar. The gas-liquid mixture formed as a result of the expansion and heating is passed through line 111 into gas-liquid separator W, from which the liquid phase obtained is discharged through line 120 for further processing. The separated gas phase is led through line 116 to medium-pressure carbamate condenser X, where a pressure preferably in the range of between about 15 and 25 bar is maintained, and condensed there with the aid of a dilute carbamate solution supplied through 117, which is obtained in further processing of the stripped urea synthesis solution. If desired, liquid ammonia can be supplied through line 118. The gases not condensed in carbamate condenser Q, consisting of inert gases, ammonia, carbon dioxide and water vapor, are discharged through line 112, and scrubbed in scrubber R, using the dilute carbamate solution obtained in medium-pressure carbamate condenser X and supplied through line 114 and pump Y. In this manner, the ammonia and carbon dioxide are recovered while the heat of absorption and condensation is removed, and the ammonia and carbon dioxide are passed through line 113 to carbamate condenser Q in the form of a carbamate solution. The remaining inert gas is vented through line 119.

## Example I

In the manner shown in Figure 1, urea is prepared in an amount of 1500 tons a day. All amounts are given in kg per hour.

Through lines 4 and 21, 34,617 kg liquid $NH_3$ is supplied to the reaction zone of reactor-stripper A and through line 28 another 800 kg liquid $NH_3$ is fed to medium-pressure carbamate condenser M. Through $CO_2$-compressor F, 45,833 kg $CO_2$ and 1,761 kg inerts (mainly air) at 120°C are supplied to the system. The pressure of this first reaction zone of reactor-stripper A is 186.3 bar and the pressure in the stripping zone is 137.3 bar. The reaction zone is further supplied through line 12 with a gas mixture from stripper D consisting of 21,064 kg $NH_3$, 25,605 kg $CO_2$, 1,786 kg $H_2O$, and 731 kg inerts, and by carbamate pump G with a carbamate solution consisting of 43,431 kg $NH_3$, 47,329 kg $CO_2$, and 10,486 kg $H_2O$. The volume of the reaction zone, and thus the residence time, is chosen so that at the chosen pressure of 186.3 bar and the corresponding temperature of about 187°C, from the streams supplied a gas-liquid mixture is formed, the liquid phase of which is composed of 51,874 kg urea, 61,188 kg $NH_3$, 29,373 kg $CO_2$, and 27,471 kg $H_2O$, while the gas phase consists of 8,529 kg $NH_3$, 5,520 kg $CO_2$, 363 kg $H_2O$, and 731 kg inerts.

In after-reactor C, also maintained at a pressure of 186.3 bar, a urea synthesis solution consisting of 64,842 kg urea, 58,478 kg $NH_3$, 22,866 kg $CO_2$, and 31,511 kg $H_2O$ is formed from the gas-liquid mixture effluent of the first reaction zone. Of this urea synthesis solution, an amount of 71,094 is treated countercurrent to a gas mixture consisting of 19,020 kg $CO_2$ and 731 kg inerts in stripper D, while the remainder, 106,643 kg, after a pressure reduction to 137.3 bar, is led to the first stripping zone in reactor-stripper A where it is treated countercurrent to a gas mixture consisting of 26,813 kg $CO_2$ and 1,030 kg inerts. In stripper D and in the stripping zone of reactor-stripper A, most of the carbamate present in the solutions supplied is decomposed, and the released $NH_3$ and $CO_2$ is expelled together with the gaseous $CO_2$ stripping gas. The heat required in stripper D is supplied by high-pressure steam of 22.6 bar. From stripper D a stripped urea solution is discharged which contains 24,770 kg urea, 10,484 kg $H_2O$, 2,988 kg $NH_3$ and 3,417 kg $CO_2$.

From the stripping zone of reactor-stripper A, a urea solution is discharged which, besides 38,710 kg urea and 16,930 kg $H_2O$, contains 10,313 kg $NH_3$ and 12,608 kg $CO_2$. The heat required for the stripping treatment is obtained by carbamate formation in the reaction zone. The gas mixture formed in the stripping zone, totaling 55,925 kg, and consisting of 24,885 kg $NH_3$, 28,068 kg $CO_2$, 1,942 kg $H_2O$, and 1,030 kg inerts gases, is introduced into carbamate condenser B where most of it is condensed with the aid of 78,044 kg of a carbamate solution (obtained from scrubbing inert gases in scrubber E) consisting of 34,002 kg $NH_3$, 35,260 kg $CO_2$, and 8,782 kg $H_2O$. The heat released in this condensation is utilized for the decomposition of carbamate still present in the stripped urea synthesis solutions obtained from stripper D and the stripping zone of reactor-stripper A. These urea synthesis solutions discharged through lines 10 and 11, are expanded to 17.7 bar. and passed through line 24 into the shell-side space of carbamate condenser B where they are passed in heat exchange with the gases condensing to carbamate in the tubes. In gas-liquid separator H, the 19,430 kg gas mixture formed in this heat exchange, consisting of 6,750 kg $NH_3$, 10,280 kg $CO_2$, and 2,400 kg $H_2O$, is separated from 100,790 kg solution containing 63,480 kg urea, 6,551 kg $NH_3$, 5,745 kg $CO_2$ and 25,014 kg $H_2O$. The gas mixture is condensed in medium-pressure carbamate condenser M and the resulting solution, totaling 40,016 kg and containing 14,788 kg $NH_3$, 16,858 kg $CO_2$ and 8,370 kg $H_2O$ is pumped to scrubber E for scrubbing of the inerts-containig gas flows supplied through lines 15 and 20. The urea solution obtained in gas-liquid separator H is passed through line 29 to a plant for further processing. In this embodiment, 18,809 kg high-pressure steam of 22.6 bar is required in stripper D, which corresponds to 301 kg per ton urea produced. No low-pressure steam is formed in carbamate condenser B.


## Example II

Using the process described, urea is prepared according to the embodiment as represented in Figure 2 in a plant having a production capacity of 1500 tonnes per day. All amounts are given in kg per hour. The pressures applied are 245 bar in reactor-stripper P, 137 bar in the tubes of carbamate condenser Q and in scrubber R, and 17.7 bar in the shell side of carbamate condenser Q, in gas-liquid separator W, and in medium pressure carbamate condenser X.

Through ejector T the reaction zone of reactor-stripper P is supplied with a gas-liquid mixture containing 34,717 kg liquid $NH_3$ and 142,949 kg of a gas mixture formed in the stripping treatment and containing 55,524 kg $NH_3$, 44,373 kg $CO_2$, 3660 kg $H_2O$, and 1,668 kg inerts. The urea synthesis solution formed in the reaction zone of reactor-stripper P is stripped in the stripping zone countercurrent to 45,833 kg $CO_2$ compressed to synthesis pressure by compressor U and introduced into the bottom of the stripping zone at a temperature of 120° C. After expansion to 137 bar, the remainder of the gas mixture obtained in the stripping treatment, consisting of 19,906 kg $NH_3$, 15,908 kg $CO_2$, 1,312 kg $H_2O$, and 598 kg inerts, is supplied to carbamate condenser Q through line 107. In addition, through line 113, 57,341 kg carbamate solution containing 22,219 kg $NH_3$, 25,021 kg $CO_2$ and 10,101 kg $H_2O$, originating from scrubber R, is supplied to carbamate condenser Q. By means of pump V, a carbamate solution consisting of 36,578 kg $NH_3$, 35,186 kg $CO_2$, 11,359 kg $H_2O$ and having a temperature of 168° C is pumped back to the reaction zone of reactor-stripper P. The non-condensed part of the gas mixture, which contains 5,547 kg $NH_3$, 5,743 kg $CO_2$, 54 kg $H_2O$, and 598 kg inerts is passed through line 112 to scrubber R.

Through line 109 from the bottom of reactor-stripper P, 126,547 kg stripped urea synthesis solution having a temperature of 175°C is discharged which, besides 63,480 kg urea and 29,091 kg $H_2O$ contains 15,417 kg $NH_3$ and 18,559 kg $CO_2$. The pressure of this solution is reduced to 17.7 bar in expansion valve 110 and the mixture obtained is passed into the shell-side space of carbamate condenser Q in heat exchange with the gas mixture condensing to carbamate in the tubes. In this heat exchange, a part of the carbamate still present in the stripped urea synthesis solution is decomposed into $NH_3$ and $CO_2$. The remaining gas-liquid mixture, with a temperature of 155°C, is subsequently supplied through line 111 to gas-liquid separator W. From the liquid-gas separator W through line 120, 101,937 kg solution containing 63,480 kg urea, 26,021 kg $H_2O$, 6,626 kg $NH_3$ and 5,810 kg $CO_2$, is discharged and sent to a plant for further processing (not shown). The gas mixture obtained in gas-liquid separator W, which contains 8,791 kg $NH_3$, 12,749 kg $CO_2$, and 3,070 kg $H_2O$, is condensed in medium-pressure condenser X, using 700 kg liquid $NH_3$, supplied through line 118, and 20,771 kg carbamate solution consisting of 7,226 kg $NH_3$, 6,568 kg $CO_2$, and 6,977 kg $H_2O$, originating from the further processing of the stripped urea synthesis solution and supplied through line 117. The carbamate solution formed in medium-pressure condenser X, having a temperature of 115°C, is pumped through line 114 to scrubber R by pump Y for scrubbing of the inerts-containing gas flow supplied through 112, which consists of 5,547 kg $NH_3$, 5,743 kg $CO_2$, 54 kg $H_2O$ and 598 kg inert gases. The inert gases are vented through line 119. In this embodiment, no high-pressure steam is required for the stripping treatment and no low-pressure steam is formed in condensation of the gas mixture obtained on stripping.

## WHAT IS CLAIMED IS:

1. In a process for the preparation of urea from carbon dioxide and an excess of ammonia at elevated pressure and temperature in at least one reaction zone to form a urea synthesis solution containing non-converted ammonium carbamate and free ammonia, which process comprises the steps of:

reacting at least a part of said ammonia and carbon dioxide to form ammonium carbamate and converting a portion of said ammonium carbamate into urea in a first reaction zone adapted to exchange heat with a first stripping zone, whereby the heat of condensation of ammonia and carbon dioxide into ammonium carbamate in said first reaction zone is exchanged with urea synthesis solution introduced into said first stripping zone so as to decompose a portion of the non-converted ammonium carbamate contained therein, and removing therefrom a gas mixture containing ammonia and carbon dioxide from a stripped urea solution still containing residual non-converted ammonium carbamate;

recycling said gas mixture, after at least partial condensation and absorption in a condensation zone, to be used in the formation of a further amount of urea synthesis solution; and

further processing said stripped urea synthesis solution to form product urea;

the improvement essentially comprising introducing said stripped urea solution, after pressure reduction, into a decomposition zone exchanging heat with said condensation zone such that heat released by said condensation is exchanged with said stripped urea solution whereby at least a portion of said residual non-converted ammonium carbamate in said stripped urea solution is decomposed.

2. The improved process according to claim 1, wherein a portion of said urea synthesis solution is introduced into a second stripping zone in which a portion of the non-converted ammonium carbamate contained therein is decomposed, and the gas mixture containing ammonia and carbon dioxide thus obtained is removed from the stripped urea solution still containing residual non-converted ammonium carbamate, and introduced into said first reaction zone wherein it is at least in part condensed to form ammonium carbamate, and stripped urea synthesis solution from said second stripping zone is introduced, together with stripped urea synthesis solution from said first stripping zone, after pressure reduction, into said decomposition zone.

3. The improved process according to claim 1, wherein the reduction in pressure of said stripped urea synthesis solution introduced into said decomposition zone is such that the pressure in said decomposition zone is at least 100 bar less than the pressure in said condensation zone.

4. The improved process according to claim 1, wherein the pressure of the stripped urea synthesis solution introduced into said decomposition zone has been reduced to between about 15 and 25 bar.

5. The improved process according to claim 2, wherein the gas mixture formed in the second stripping zone is condensed and at least in part converted into a urea-containing ammonium carbamate solution in said first reaction zone.

6. The improved process according to claim 2, wherein at least a portion of the carbon dioxide required for the urea preparation is fed to the second stripping zone as a stripping agent, thereby enriching the gas mixture formed therein with respect to carbon dioxide.

7. The improved process according to claim 6, wherein said carbon dioxide enriched gas mixture formed in the second stripping zone, as well as ammonia, is supplied to said first reaction zone.

8. The improved process according to claim 6, wherein a portion of the carbon dioxide required for the urea preparation is fed to the first stripping zone.

9. The improved process according to claim 6, wherein between about 30 and 50 percent of the carbon dioxide required for the urea preparation is fed to the second stripping zone.

10. The improved process according to claim 2, wherein between about 40 and 60 percent of the urea synthesis solution formed in said second reaction zone is fed to said second stripping zone.

1/2

FIG. 1

0136765

FIG. 2